# EUROPEAN PATENT APPLICATION

(11) **EP 0 747 080 A1**
(43) Date of publication of application: **11.12.1996**
(21) Application number: 96304276.7
(22) Date of filing: 06.06.1996
(51) Int. Cl.: A61M 21/00, H04R 1/10

(54) **Brain wave inducing device**

(30) Priority: 08.06.1995 JP 141774/95
(71) Applicant: ALPHA INTERNATIONAL CORP., Shinjuku-ku, Tokyo (JP)
(72) Inventor: Sawafuji, Tadashi, Tokyo (JP)
(74) Representative: Watkins, David

(57) **Abstract**

A brain wave inducing device includes an oscillator (1) for generating sine waves which are components of brain wave signals and a plane wave speaker (2) connected to the output side of the oscillator (1). The plane wave speaker (2) may be incorporated in a headphone (7).

## Description

The present invention relates to a brain wave inducing device for directly inducing brain waves such as alpha waves.

The relation between brain waves and mental activities has become apparent, and it has been known that the brain waves influence the mind and body in accordance with the change of brain wave signals. That is, delta waves with a frequency of 0.4 to 4 Hz correspond to the state of deep sleep, theta waves with a frequency of 4 to 8 Hz correspond to the state of shallow sleep, alpha waves with a frequency of 8 to 14 Hz correspond to the sate of relaxation, beta waves with a frequency of 14 to 28 Hz correspond to the state of tension, and gamma waves with a frequency of more than 28 Hz correspond to the state of excitement.

For example, if the brain waves are made into the state of alpha waves, memories formed in this state enter into the subconscious. What is learned in this state is reproduced through the alpha waves, so that ability is developed and energy, powers of concentration, powers of retention, powers of judgement, powers of creation, powers of observation, powers of persuasion, powers of expression, powers of thought, vitality and the like are strengthened.

Thus, there have conventionally been various proposals to make brain waves into alpha waves, or to induce theta waves or delta waves. As stimulants for inducing brain waves, there are the sense of hearing, the sense of sight, the sense of touch, the sense of smell, the sense of taste, and the like. As an example of a device using the sense of hearing to induce brain waves, there is a device comprised of an oscillator for generating two low frequency electric signals having a frequency difference of about 4 to 16 Hz and sound generating means for converting those low frequency electric signals, which are within an audible range, into an audible sound including a beating sound having the frequency difference (Japanese Patent Publication No. Hei. 2-22674).

According to the above brain wave inducing device comprised of the sound generating means, two low frequency electric signals having a frequency difference of about 4 to 16 Hz are converted into an audible sound including a beating sound having this frequency difference. Brain waves with a frequency from about 4 to 16 Hz are indirectly induced by hearing the sound. As a result, it takes a long time to induce the brain waves. Further, in this device, the sound is always heard during use thereof, so that there is a likelihood that a user would experience difficulty in performing intellectual work such as study while using this device.

In view of the above, an object of the present invention is to provide a brain wave inducing device which is intended to enable brain waves to be easily induced and to shorten the time necessary for the inducement by directly inducing the brain waves through sine waves of brain wave signals, and which is further intended to enable intellectual work or the like to be easily performed at the same time as the inducement and also to enable sound such as music to be heard during the inducement by inducing the brain waves in the soundless - or almost soundless - state.

In order to achieve the above object, a plane wave speaker is connected to the output side of an oscillator for generating sine waves of components of the brain waves.

According to this structure, the sine waves generated from the oscillator are enlarged by the plane wave speaker and are output as plane sound waves with the same frequency as the sine waves. These plane sound waves have no diffused reflection, and one periodic wave is accurately transmitted, as one push and pull, to the eardrum of a listener so that the plane sound waves accurately deform and move the eardrum. The deformation movement of the eardrum interlocks the malleus, incus and stapes, and the cerebrum is stimulated from the cochlea through the cochlea nerve. As a result, brain waves are induced having the same frequency as that of the sine waves generated from the oscillator.

Also, according to the present invention, the plane wave speaker may be built into a headphone.

By putting the headphone with the built-in plane wave speaker on the ear, the ear is closed by the headphone so that the external auditory meatus becomes almost sealed. In this state, when the plane sound waves are output from the plane wave speaker, the air in the external auditory meatus is subjected to parallel positive pressure and negative pressure by the sound pressure due to one push and pull of one periodic wave, and this positive and negative pressure of the air deforms the eardrum. Thus, deformation movement of the eardrum by the plane sound waves is more accurately and certainly performed. This deformation movement of the eardrum interlocks the malleus, incus and stapes, and the cerebrum is stimulated from the cochlea through the cochlea nerve, whereby brain waves having the same frequency as that of the sine waves generated from the oscillator are more efficiently induced.

Preferred embodiments of the present invention will now be described in detail with reference to the drawings, in which:
Figure 1 is a block diagram showing an embodiment of the present invention;
Figure 2 is a schematic sectional view showing an example of a plane wave speaker;
Figure 3 is a construction view showing another embodiment of the present invention, and
Figure 4 is a sectional view showing an example of a headphone with a built-in plane wave speaker.

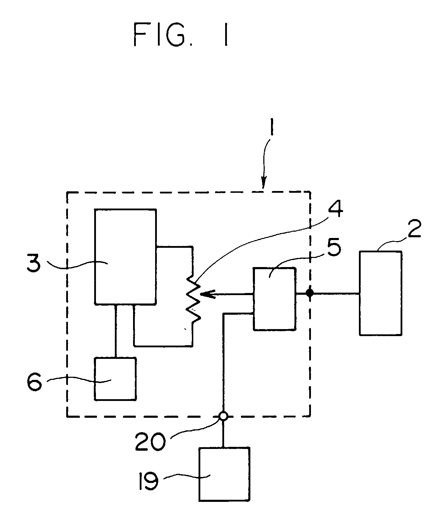

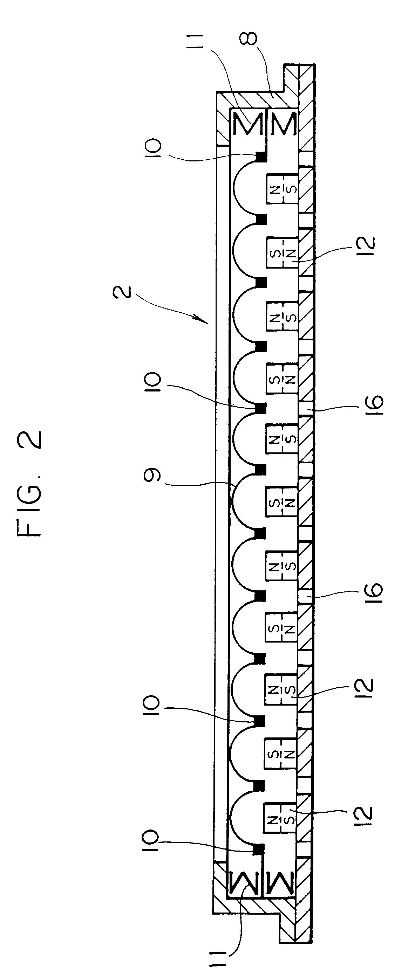

Figures 1 and 2 show an embodiment of the present invention. Figure 1 is a block diagram showing the embodiment and Figure 2 is a schematic sectional view showing an example of a plane wave speaker.

In the figures, reference numeral 1 denotes a CR oscillator for generating sine waves that are components of brain waves, and 2 denotes a plane wave speaker connected to the output side of the CR oscillator.

The CR oscillator is comprised of a sine wave generating circuit 3, a frequency adjuster 4, an OP amplifier 5 and a timer 6. In this embodiment, the sine wave generating circuit 3 arbitrarily and selectively generates sine waves, which are components of brain wave signals having frequencies known generally as alpha waves, theta waves, and delta waves. However, the circuit is not limited to the above-mentioned one. The circuit may be such that only sine waves of a specified frequency, for example the frequency range of alpha waves, are generated.

Among the above-mentioned brain wave signals, the brain wave signals known as alpha waves appear together with the rhythm of fluctuation. Thus, in this embodiment, the frequency of brain waves of alpha waves is detected, which is analyzed to be stored in a memory, and this memory is contained in the sine wave generating circuit 3, so that sine waves with the frequency of alpha waves accompanied with the rhythm of fluctuation similar to the brain waves are generated.

The plane wave speaker 2 connected to the output side of the CR oscillator 1 having the above described structure, is a speaker based on the principle that a plane sound wave is generated by driving a flat oscillating plate at the same phase by the input of sound signals. Such a principle is well known, for example, as exemplified in a sound device disclosed in Japanese Patent Publication No. Sho. 58-237. A speaker based on this principle is put into practical use.

The plane wave speaker 2 shown in Figure 2 is constructed as follows:

Reference numeral 8 denotes a case, and 9 denotes an oscillating plate formed of a hard and light material. A voice coil 10 is mounted on the oscillating plate 9 which is supported by the case 8 through an edge 11. The entire surface of this oscillating plate 9 is driven in parallel by a magnetic circuit formed of a magnet 12. A vent hole 16 is formed in the rear surface of the case 8.

Reference numeral 19 denotes an external sound source device such as a CD player or a tape player, which is connected to the oscillator 1 through an external terminal 20 and which can be superimposed on the sound signals generated from the sine wave generating circuit 3.

The operation of the above embodiment will next be described:

The plane wave speaker 2 is positioned in a living space which is relatively airtight in comparison to the open air, such as a house, an apartment, a hotel or an office.

By the sine wave generating circuit 3 of the oscillation circuit 1, a particular pattern of sine waves, which are components of brain wave signals having frequencies generally designated as alpha waves, theta waves, delta waves and the like, is selectively generated. The pattern or frequency range of sine waves selected will depend on the type of brain waves which it is intended to induce. These sine waves are amplified by the OP amplifier 5 and input as sound signals into the plane wave speaker 2. The sound signals input into the plane wave speaker 2 are excited and enlarged, then drive the flat oscillating plate 9 in parallel, and are output as plane sound waves with the same frequency.

These plane sound waves have no diffused reflection, and one periodic wave is accurately transmitted, as one push and pull, to the eardrum of a listener so that the plane sound waves accurately deform and move the eardrum. This deformation movement of the eardrum interlocks the malleus, incus and stapes, and the cerebrum is stimulated from the cochlea through the cochlea nerve. As a result, brain waves are induced having the same frequency as the sine waves generated by the oscillator 1.

At this time, since the cerebrum is directly stimulated by sound waves with the same frequency as that of the brain waves such as alpha waves, theta waves, gamma waves, and the like, the inducement of brain waves having the same frequency can be carried out in a short time and with ease.

According to experiments, when sine waves of 8 to 14 Hz were oscillated by the oscillator 1, in many experiments it was detected that the brain waves became full of alpha waves in about 5 minutes. In comparative examples, using indirect inducement by audible sound, it took 10 to 20 minutes for the brain waves to become full of alpha waves.

As mentioned above, the plane wave speaker 2 outputs plane sound waves having the same frequency as that of alpha waves, theta waves, delta waves and the like. Since these frequencies are low frequencies, the sound waves are inaudible sounds or almost inaudible sounds. Thus, a user rarely feels the sense of using a device. For example, when the device is used to induce brain waves of alpha waves, it is possible easily to perform intellectual work such as study while using this device. Further, since the intellectual work is performed under the state in which brain waves of the alpha type are always induced, the efficiency of the work is remarkably improved.

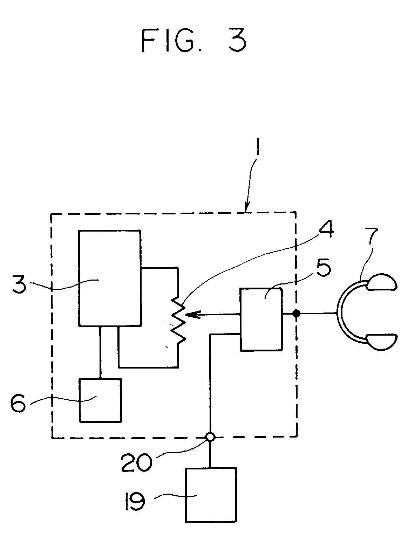

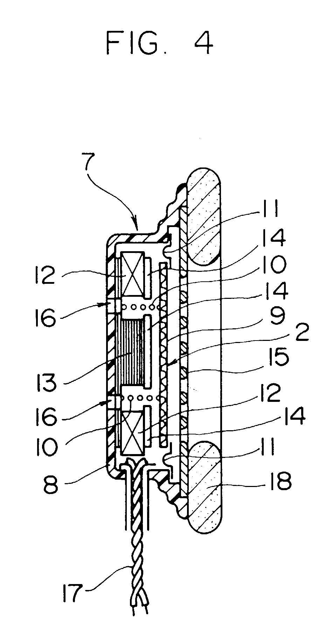

Figures 3 and 4 show another embodiment of the present invention. In this embodiment, the plane wave speaker 2 described before is built into a headphone 7. Figure 3 is a block diagram showing this embodiment, and Figure 4 is a sectional view showing an example of the headphone with the built-in plane wave speaker 2.

The head-phone 7 shown in Figure 4 is constructed as follows:

Reference numeral 8 denotes a case, and 9 denotes a flat oscillating plate formed of a hard and a lightweight material. A voice coil 10 capable of performing a long stroke is especially mounted to the oscillating plate 9 which is supported by the case 8 through an edge 11. The entire surface of this oscillating plate 9 is driven in parallel by a magnetic circuit formed of a magnet 12.

Reference numeral 13 denotes a yoke, 14 denotes a pole, 15 denotes a net, 17 denotes a cord, and 18 denotes an ear pad.

The operation of the embodiment described above will next be described:

The headphones 7 are place on both ears while, by the sine wave generating circuit 3 of the oscillator 1, a particular pattern of sine waves which are components of brain wave signals having a frequency generally designated as alpha waves, theta waves, delta waves and the like, is selectively generated. The pattern or frequency range of sine waves selected will depend on the type of brain waves which it is intended to induce. The sine waves are input as sound signals into the plane wave speaker 2 of the headphone 7. The sound signals input into the plane wave speaker 2 are excited and enlarged to drive the flat oscillating plate 9 in parallel, so that the plane sound waves of the same frequency are output into the external auditory meatus.

At this time, the ears are blocked by the headphones 7, and the external auditory meatus is almost sealed. When the plane sound waves are output into the external auditory meatus in this state, the air in the external auditory meatus is subjected to parallel positive pressure and negative pressure by sound pressure due to one push and pull of one periodic wave. The positive and negative pressure of the air deforms the eardrum, so that deformation movement of the eardrum by the plane sound waves is performed more accurately and certainly. This deformation movement of the eardrum interlocks the malleus, incus and stapes, and the cerebrum is stimulated from the cochlea through the cochlea nerve. According to this, brain waves having the same frequency as the sine waves oscillated from the oscillator 1 are induced more efficiently.

In the above described embodiments of the present invention, an external sound source device 19 such as a CD player or a tape player is connected to the oscillator 1 through an external terminal 20, and can be superimposed on the sound signals generated from the sine wave generating circuit 3. Thus, by producing and superimposing music and other sounds which can indirectly induce brain waves that are directly induced by sound signals generated by the sine wave generating circuit 3, a user can hear only the sound generated by the external sound source device 19. This sound and the sound waves based on the sound signals generated by the external sound source device 19, for example by the sine wave generating circuit 3, produce multiplier effects, so that predetermined brain waves are more efficiently induced.

As described above, according to the brain wave inducing device of the present invention, a plane wave speaker is connected to the output side of an oscillator for generating sine waves which are components of the brain waves. Thus, by directly stimulating the brain with sound waves having the same frequency as brain waves to be induced, it becomes possible to induce brain waves having the same frequency. According to this, it is possible to induce brain waves having the same frequency in a short time and with great ease.

Further, since the frequency of brain waves such as alpha waves, theta waves, delta waves and the like, which are to be induced, is a low frequency, the sound wave having the same frequency output from the plane wave speaker is soundless (inaudible sound) or almost soundless. As a result, a user rarely feels the sense of using a device. For example, when the device is used to induce brain waves of alpha waves, intellectual work such as study can be easily performed while using this device. Further, since the intellectual work is performed in the state in which the alpha waves are always induced, there is obtained the effect that the efficiency of the work can be remarkably improved.

## Claims

1. A brain wave inducing device, comprising:
an oscillator (1) for generating sine waves which are components of brain wave signals, and
a plane wave speaker (2) connected to an output side of said oscillator (1).

2. A brain wave inducing device as claimed in claim 1 further comprising a headphone (7), said plane wave speaker (2) being built into said headphone (7).
